# EUROPEAN PATENT SPECIFICATION

(11) **EP 3 599 614 B1**
(45) Date of publication and mention of the grant of the patent: **25.08.2021**
(21) Application number: 18185044.7
(22) Date of filing: 23.07.2018
(51) Int. Cl.: G16H 40/63

(54) **MEDICAL APPARATUS SYSTEM FOR CHARGING A MOBILE COMPONENT OF A MEDICAL APPARATUS**
MEDIZINISCHES VORRICHTUNGSSYSTEM ZUM LADEN EINER MOBILEN KOMPONENTE EINER MEDIZINISCHEN VORRICHTUNG
SYSTÈME D'APPAREIL MÉDICAL POUR CHARGER UN COMPOSANT MOBILE D'UN APPAREIL MÉDICAL

(43) Date of publication of application: 29.01.2020
(73) Proprietor: TRUMPF Medizin Systeme GmbH + Co. KG, 07318 Saalfeld (DE)
(72) Inventor: FIOLKA, Adam, Batesville, Indiana 47006 (US); CZEKANOWSKI, Paul, Batesville, Indiana 47006 (US); DETERT, Martina, Batesville, Indiana 47006 (US)
(74) Representative: Prüfer & Partner mbB Patentanwälte · Rechtsanwälte

(56) References cited:
- US-A1- 2006 265 807
- US-A1- 2010 314 946
- US-A1- 2012 117 730
- US-A1- 2015 061 897
- US-A1- 2016 229 301
- US-B1- 8 398 408

## Description

The invention relates to a medical apparatus system, in particular to a medical apparatus system comprising a mobile component of a medical apparatus provided with an integrated accumulator.

Heretofore, mobile medical apparatuses, as, e.g., operating tables, having an integrated accumulator for providing electric power for a controller and electric actuators of the medical apparatuses have already been known. The accumulators of these mobile medical apparatuses have usually been charged via a power cord connected to the medical apparatus and a socket of a power supply.

However, in particular, when the medical apparatus is used during a surgical intervention and the power cord is used for ensuring a function of the medical apparatus during the surgical intervention, there is a risk that the medical personnel trips over the power cord so that dangerous situations for the medical personnel and the patient can occur. Moreover, in case that connecting the medical apparatus and the power supply has been forgotten, there was the risk that the medical apparatus stops functioning during the surgical intervention and/or that the connection has to be established during the surgery which is accompanied with hygienic risks.

Document US 2012/011 77 30 A1 discloses a patient support apparatus including the features of the preamble of claim 1.

Therefore, the object underlying the invention is to avoid these disadvantages and to provide a medical apparatus system ensuring a sure and comfortable power supply of the mobile medical apparatus.

The object is achieved by a medical apparatus system according to claim 1 and a method according to claim 7.

According to an aspect of the invention, a medical apparatus system comprises a mobile component of a medical apparatus comprising an integrated accumulator for providing electric power for a control device and for electric actuators of the mobile component of the medical apparatus, and an interface configured to automatically connect a power supply device and the accumulator only at a stationary connecting location. The interface comprises a room-side interface component and a collaborating medical apparatus-side interface component.

By such a medical apparatus system, a power cord-less connection of the integrated accumulator and the power supply device at the stationary connecting location is possible such that the risks that, on the one hand, the medical personnel trips over the power cord and, on the other hand, due to the automatic connection, connecting the mobile component of the medical apparatus and the power supply device can been forgotten are eliminated and a safe and comfortable operation of the mobile medical apparatus is enabled. Due to the stationary connecting location, there is no need to provide an extensive room-side interface component as e.g. contact rails. The mobile component of the medical apparatus can be formed by an entire medical apparatus or by a single mobile component of the medical apparatus or a mobile component joint to the medical apparatus, e.g. a shuttle.

In an advantageous implementation of the medical apparatus system, the room-side interface component and the medical apparatus-side interface component respectively comprise inductively transmitting components configured to interact for transmitting electric power from the power supply device to the accumulator.

In case of the inductively transmitting components of the interface, no plug-in connection are present so that the risk of contamination or damaging such that enabling an electric contact is not possible any more is eliminated.

In a further advantageous implementation of the medical apparatus system, the room-side interface component comprises an underfloor component and the apparatus-side interface component is located in a support structure of the mobile component of the medical apparatus.

By providing the room-side interface component as the underfloor component, i.e., the location of the room-side interface component in the floor, a barrier-free access to a working position of the mobile component of the medical apparatus is enabled. Furthermore, a flat floor is possible and there are no edges or gaps which include the risk of contamination. When, additionally, the apparatus-side interface component is located in a support structure of the mobile component of the medical apparatus, the transmission of the electric power can be easily performed.

In a further advantageous implementation of the medical apparatus, the room-side interface component comprises at least one of a wall component being arranged in a wall of a predefined room, a charging adapter in a separate housing being arranged on a floor or at the wall of the predefined room, and a charging mat located on the floor of the predefined room.

Due to the provision of the room-side interface component comprising the wall component, the charging adapter or the charging mat, advantageous charging the accumulator is possible according to local circumstances and, as the case may be, without huge installation efforts.

In another advantageous implementation of the medical apparatus system, the room-side interface component and the medical apparatus-side interface component respectively comprise a component of a plug-in connection configured to interact for transmitting electric power from the power supply device to the accumulator, wherein the medical apparatus system is configured to automatically connect the room-side interface component and the medical apparatus-side interface component by an initiation signal.

When comprising a plug-in connection, the connection has a larger performance for charging the accumulator and for directly energizing the actuators and the control device of the mobile component of the medical apparatus. The connection is automatically performed by an initiation signal which can be generated by the mobile component of the medical apparatus system itself, e.g. when a sensor signals that the components are aligned, or by a manual signal when an operator initiates the connection.

In a further advantageous implementation of the medical apparatus system, the medical apparatus system comprises marking components, guiding components or docking components attachable on the floor of the predefined room for positioning the operating table-side interface component of the mobile operating table with respect to the room-side interface component.

When providing marking, guiding or docking components for the positioning of the operating table-side interface component, the positioning of the mobile component of the medical apparatus is further facilitated and a sure power transmission is ensured.

In a further advantageous implementation of the medical apparatus system, the room-side interface component comprises a charging electronics configured to control the charging operation.

By equipping the room-side interface component with the charging electronics, power transmission can be controlled in an easy and compact manner without providing long sensor lines for transmitting signals of a state of the mobile component of the medical apparatus.

In a further advantageous implementation of the medical apparatus system, the charging electronics is configured to automatically start the charging operation after a connection of the room-side interface component and the medical apparatus-side interface component.

By this configuration, the power transmission can start as soon as possible, the connection can be established comfortably, and connecting cannot be forgotten such that there is no risk that the accumulator runs out of power during operation.

In a further advantageous implementation of the medical apparatus system, the medical apparatus comprises an operating table.

By using the operating table as the mobile component of the medical apparatus, a position of a patient placed on the operating table before a surgical invention, e.g. during the carriage, can be easily altered in an electrically driven manner without being connected to a socket, e.g., in case of an emergency.

In a further advantageous implementation of the medical apparatus system, the medical apparatus comprises a lamp.

When the lamp, e.g., an examination lamp or an operating lamp, is used as the component of the mobile medical apparatus, at least for a short-term use, the lamp can be flexibly positioned at a suitable location for individually illuminating an arbitrary field and, in case of a power blackout, a continuous illumination is ensured in a simple manner.

In a further advantageous implementation of the medical apparatus system, the operating table and the surgical lamp are alternative components of medical apparatuses respectively comprising the medical apparatus-side interface component configured to collaborate with the identical room-side interface component.

Due to the use of such a medical apparatus system, the accumulators of the operating lamp and of the surgical table can alternately be charged by means of the identical room-side interface component without the need of multiple and/or different room-side interface components.

According to another aspect of the invention, a method for charging the accumulator of the mobile component of the medical apparatus of the medical apparatus system comprises the steps: positioning the medical apparatus-side interface component of the mobile component of the medical apparatus with respect to the room-side interface component, and charging the accumulator of the mobile component of the medical apparatus by transmitting electric power via the medical apparatus-side interface component and the room-side interface component.

By this method, a power cord-less connection of the accumulator and the power supply device is possible such that the risks that, on the one hand, the medical personnel trips over the power cord and, on the other hand, connecting the mobile component of the medical apparatus and the power supply device can be forgotten are eliminated and a safe and comfortable operation of the mobile component of the medical apparatus is enabled.

In an advantageous implementation, the method includes the step of automatically starting charging of the accumulator after connecting the interface components.

By this step, on the one hand, charging starts as early as possible, nevertheless, without the risk of damaging electronic components due to voltage peaks, on the other hand, charging of the accumulator cannot be forgotten.

In an advantageous implementation of the method, the positioning is performed by aligning specific features of the mobile component of the medical apparatus and markings, guiding components or docking components on a floor of the predefined room.

When aligning specific features of the mobile table, e.g. a lateral edge and a front edge, with markings or guiding components on the floor or other specific features, e.g. dockable components, with the docking components on the floor, the positioning of the mobile operating table is facilitated and a sure power transmission is ensured.

In a further advantageous implementation of the method, the charging is performed by an inductive connection of the interface components.

When performing an inductive power transmission for the charging, no plug-in connection are present so that the risk of contamination or damaging such that enabling an electric contact is not possible is eliminated.

In a further advantageous implementation of the method, the charging is performed by a plug-in connection of the interface components.

When charging is realized via a plug-in connection, the connection has a large performance for charging the accumulator and for directly energizing the drives and the control device of the component of the movable medical apparatus.

Now, the invention is elucidated by means of embodiments referring to the attached drawings.

In particular:
- Fig. 1: shows a principle illustration of an operating table system as a medical apparatus system of a first example;
- Fig. 2: shows a principle illustration of a further operating table system as a medical apparatus system of an embodiment according to the invention;
- Fig. 3: shows a principle illustration of alternatives of the operating table system as the medical apparatus system of a second example;
- Fig. 4: shows a principle illustration of a further alternative of the operating table system as the medical apparatus system of the first example;
- Fig. 5: shows a principle illustration of another operating table system as a medical apparatus system of a third example;
- Fig. 6: shows a principle illustration of a lamp system as a medical apparatus system of a fourth example, and
- Fig. 7: shows a flowchart of a method for charging an accumulator of a mobile component of a medical apparatus of the medical apparatus system according to any embodiment of the invention.

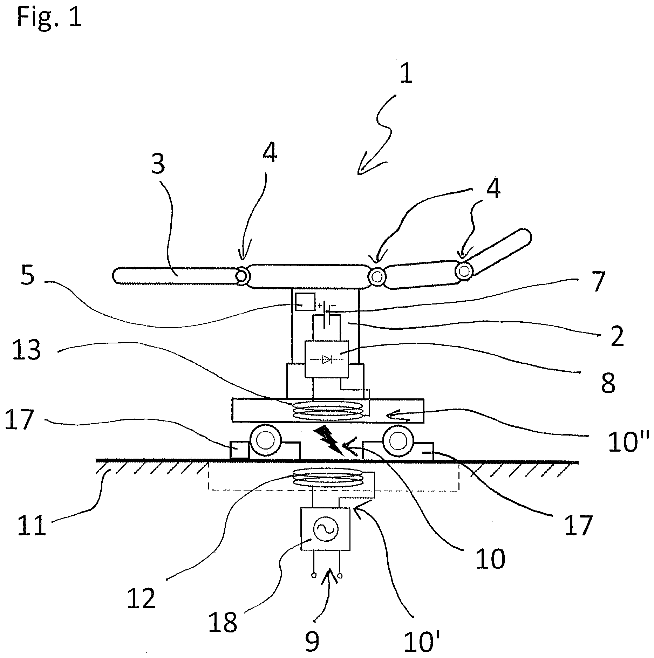

Fig. 1 shows a principle illustration of an operating table system as a medical apparatus system 1 of a first example.

The medical apparatus system 1 comprises a mobile component of a medical apparatus 2 in the form of a mobile operating table provided with castors for being movable. The mobile component of the medical apparatus 2 comprises a patient support plate 3 including swivel joints respectively joining several sections of the patient support plate 3. The sections can be swiveled by electric drives as electric actuators 4. Furthermore, the mobile component of the medical apparatus 2 comprises a control device 5 for controlling the electric actuators 4 and an integrated accumulator 7 for providing electric power for the control device 5 and for the electric actuators 4. The accumulator 7 is provided with a charging controller 8. The charging controller 8 controls charging of the accumulator 7 depending on an available power supply device 9 and the state of charge of the accumulator 7. Alternatively, the mobile component of the medical apparatus can also be formed by a shuttle joined to the medical apparatus for moving a tabletop or for moving a tabletop attached to an operating table column.

The accumulator 7 of the mobile component of the medical apparatus 2 is automatically connected to the power supply device 9 only at a stationary connecting location. For establishing the connection, the medical apparatus system 1 comprises an interface 10 for connecting the power supply device 9 and the accumulator 7. The interface 10 comprises a room-side interface component 10' at a stationary connecting location and a collaborating medical apparatus-side interface component 10". Automatically connected means that, besides positioning the mobile component of the medical apparatus 2 and, as the case may be, initiating a coupling motion, there is no further action, e.g. connecting by a power cord, necessary for connecting the supply device 9 and the accumulator 7.

The room-side interface component 10' is an underfloor component and it is located in a floor 11 of a predefined room. Alternatively, depending on a type of the mobile component of the medical apparatus 2, the room-side interface component 10' can be located in a wall of the predefined room, in a pedestal or can be movably located in the predefined room.

In the first example of the medical apparatus system 1, the room-side interface component 10' and the medical apparatus-side interface component 10" respectively comprise inductively transmitting components interacting for transmitting electric power from the power supply device 9 to the accumulator 7. In this example, the inductively transmitting components of the room-side interface component 10' comprise a first induction coil 12 and the inductively transmitting components of the medical apparatus-side interface component 10" comprise a second induction coil 13. The second induction coil 13 is arranged in a bottom portion of the mobile component of the medical apparatus 2.

The medical apparatus system 1 further comprises guiding components 17 on the floor 11 of the predefined room for positioning the medical apparatus-side interface component 10" of the mobile component of the medical apparatus 2 with respect to the room-side interface component 10'. The guiding components 17 are attached to the floor 11 for guiding the castors of the mobile component of the medical apparatus 2 such that, at the stationary connecting location, a position of the medical apparatus-side interface component 10" matches with a position of the room-side interface component 10'. In alternative embodiments, instead of or additionally to the guiding components 17, marking components for visibly guiding the mobile component of the medical apparatus or docking components for locking the mobile component of the medical apparatus at the stationary connecting location are possible.

The room-side interface component 10' comprises a charging electronics 18 controlling the charging operation. The charging electronics is integrated in the room-side interface component 10'; nevertheless, alternatively, it can be separately provided. In a specific embodiment, the charging electronics 18 is configured to automatically start the charging operation after a connection of the room-side interface component 10' and the medical apparatus-side interface component 10".

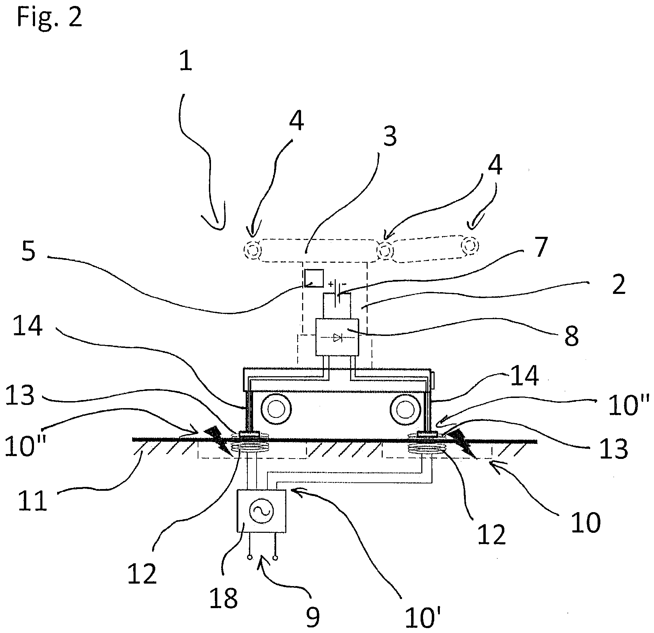

Fig. 2 shows a principle illustration of a further operating table system as a medical apparatus system 1 of an embodiment according to the invention. The medical apparatus system 1 according to the embodiment differs from the medical apparatus system 1 according to the first example in the quantity and the location of the first and second induction coils 12, 13.

The operating table as the mobile component of the medical apparatus 2 according to the embodiment of the invention is also provided with castors for being movable; however, in the embodiment, the operating table is also provided with jack up stamps as support structures 14 for securing a sure footing of the mobile component of the medical apparatus 2. Moreover, respectively two first and second induction coils 12, 13 are provided. In alternative embodiments, only respectively one first and second induction coil 12, 13 or more than respectively two first and second induction coils 12, 13 are provided.

As in the first example, the first induction coils 12 of the inductively transmitting components of the room-side interface component 10' are located in the floor 11 of the predefined room, whereas the second induction coils 13 are arranged at the support structures 14. Optionally, the support structures 14 can be provided with a switch for detecting a contact of the support structure 14 with the floor 11 or, alternatively with an alternative housing of the room-side interface component 10'.

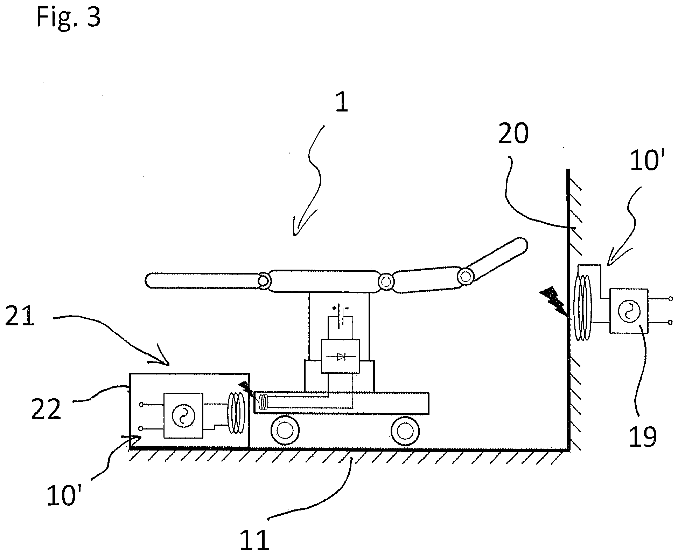

Fig. 3 shows a principle illustration of alternatives of the operating table system as the medical apparatus system 1 of a second example. According to one alternative, the room-side interface component 10' comprises a wall component 19 being arranged in a wall 20 of the predefined room.

According to another alternative, the room-side interface component 10' comprises a charging adapter 21 in a separate housing 22 being arranged on a floor 11 of the predefined room. In a further alternative, the charging adapter 21 in the separate housing 22 can also be arranged at the wall 20 of the predefined room.

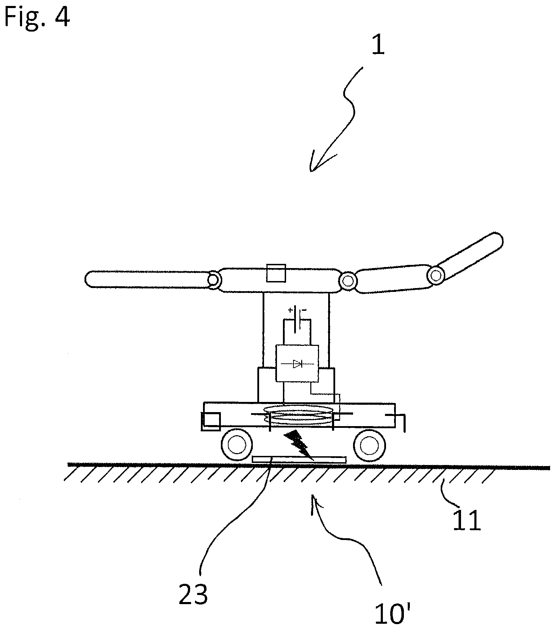

Fig. 4 shows a principle illustration of a further alternative of the operating table system as the medical apparatus system 1 of the first example. In this alternative, the room-side interface component 10' comprises a charging mat 23 located on the floor 11 of the predefined room.

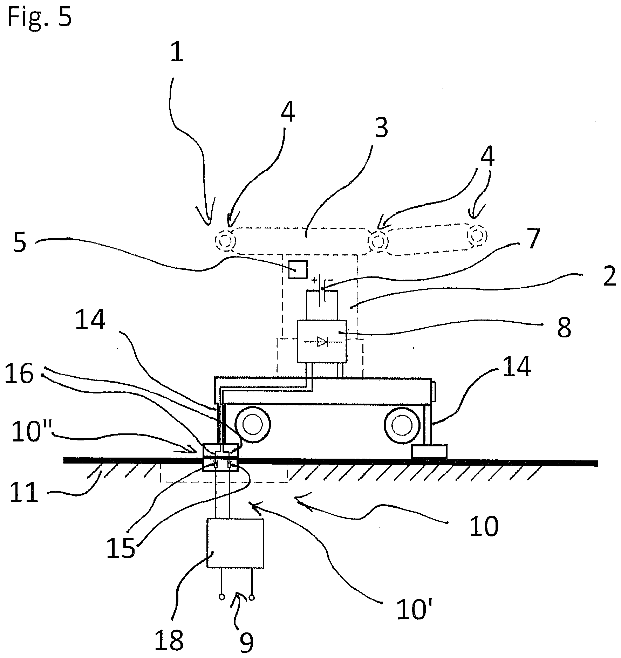

Fig. 5 shows a principle illustration of another operating table system as a medical apparatus system 1 of a third example according to the invention. The medical apparatus system 1 according to the third example differs from the medical apparatus system 1 according to the embodiment in the physical principle of the interface.

In the third example, the room-side interface component 10' and the medical apparatus-side interface component 10" respectively comprise a component of a plug-in connection interacting for transmitting electric power from the power supply device 9 to the accumulator 7. In this example, the room-side interface component 10' is also located in floor 11. However, the options concerning the location of the room-side interface component 10' of the other embodiments are also appropriate in this embodiment.

The room-side interface component 10' includes sockets 15 for providing the electric power and the medical apparatus-side interface component 10" includes plug connectors 16 for being inserted into the sockets 15. The electric contact is established by lowering the support structures 14 such that the sockets 15 and the plug connectors 16 are connected. Alternatively, the sockets 15 and the plug connectors 16 are, at least partially, interchanged or at other locations of the mobile component of the medical apparatus 2 and in the predefined room. Furthermore, alternatively, another quantity of the room-side interface components 10' and of the medical apparatus-side interface components 10" is possible.

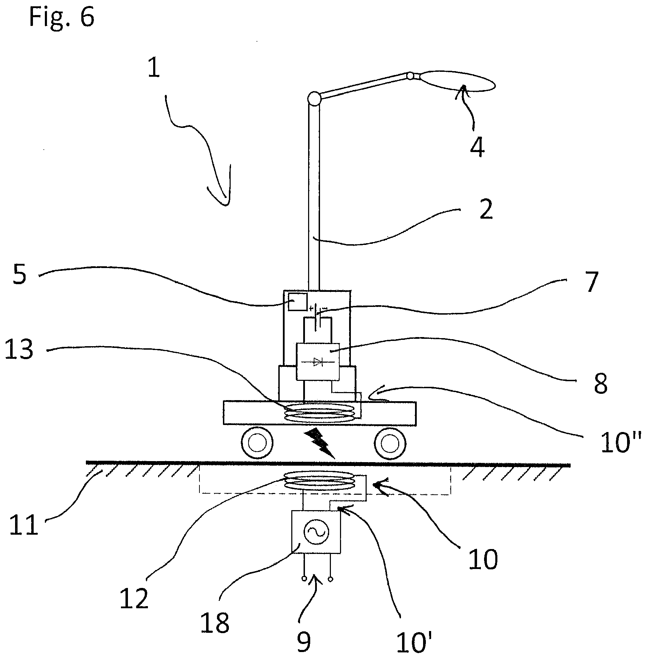

Fig. 6 shows a principle illustration of a lamp system as a medical apparatus system 1 of a fourth example. The medical apparatus system 1 according to the fourth example differs from the medical apparatus system 1 according to the embodiment in type of the mobile component of the medical apparatus 2. In this embodiment, the mobile component of the medical apparatus 2 is a lamp. The lamp can, e.g., be an examination light or a surgical light.

In this embodiment, the actuators 4 are illuminants for illuminating an examination site or a surgical site. The other components, except the table-specific components, correspond to the components according to the first embodiment.

In alternative embodiments, the medial apparatus systems 1 according to the first embodiment and according to the first to fourth example can comprise the lamp as the mobile component of the medical apparatus 2 and, moreover, the medical apparatus systems 1 can respectively include another mobile component of the medical apparatus as long as it is supplied by an integrated accumulator.

As to be seen from Fig. 1 and Fig. 6, the operating table and the lamp are alternative mobile components of the medical apparatuses respectively comprising the medical apparatus-side interface component 10" configured to collaborate with the identical room-side interface component 10'. In alternative embodiments, the medical apparatus-side interface components 10" interact with different room-side interface components 10'.

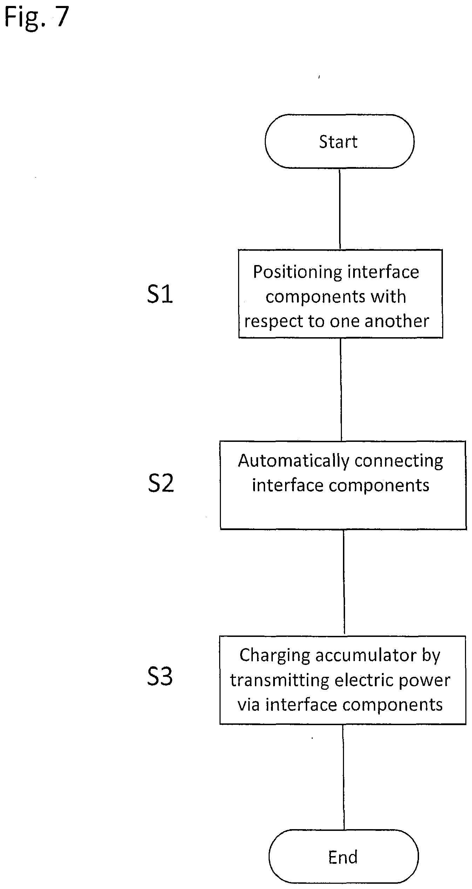

Fig. 7 shows a flowchart of a method for charging the accumulator 7 of the mobile component of the medical apparatus 2 of the medical apparatus system 1 according to any embodiment of the invention.

In use, in the method for charging the accumulator 7 of the mobile component of the medical apparatus 2 of the medical apparatus system 1, in Step S1, the medical apparatus-side interface component 10" of the mobile component of the medical apparatus 2 is positioned with respect to the room-side interface component 10' and simultaneously or subsequently, in Step S2, the room-side interface component (10') and the medical apparatus-side interface component (10") are automatically connected. Subsequently, in Step S3, the accumulator 7 of the mobile component of the medical apparatus 2 is charged by transmitting electric power via the room-side interface component 10' and the medical apparatus-side interface component 10". Optionally, the charging starts automatically after connecting the interface components. Connecting means that a function of an inductive coupling or of an electromechanical coupling is has been established.

When positioning the medical apparatus-side interface component 10" of the mobile component of the medical apparatus 2 with respect to the room-side interface component 10', if available, specific features of the mobile component of the medical apparatus 2 and markings, guiding components 17 or docking components on the floor 11 of the predefined room are aligned.

Depending on the provided physical principle, the charging is performed by an inductive connection of the interface components 10', 10" or by a plug-in connection of the interface components 10', 10".

On the one hand, the predefined room can be an operating theater, wherein the function of the mobile component of the medical apparatus is ensured since running out of electric power due to an uncharged accumulator is prevented. On the other hand, the predefined room can also be another room, e.g., a preparation room where the patient is prepared for a surgical intervention. Furthermore, the accumulator of the mobile component of the medical apparatus can be charged in a specific room or at a specific location where the mobile component of the medical apparatus is cleaned or preserved until a next usage.

In the claims, the word "comprising" does not exclude other elements or steps, and the indefinite article "a" or "an" does not exclude a plurality.

## Claims

1. A medical apparatus system (1) comprising
a mobile component of a medical apparatus (2) comprising an integrated accumulator (7) for providing electric power for a control device (5) and for electric actuators (4) of the mobile component of the medical apparatus (2), and
an interface (10) configured to automatically connect a power supply device (9) and the accumulator (7) only at a stationary connecting location, wherein
the interface (10) comprises a room-side interface component (10') and a collaborating medical apparatus-side interface component (10"),
wherein
the room-side interface component (10') comprises an underfloor component and one or more first induction coils located in a floor, and
the apparatus-side interface component (10") is located in support structures (14) of the mobile component of the medical apparatus (2),
**characterized in that**
the mobile component of the medical apparatus (2) comprises jack-up stamps as the support structures (14) for securing a safe footing of the mobile component of the medical apparatus (2),
one or more second induction coils (13) are arranged at the support structures (14).

2. The medical apparatus system (1) of claim 1, wherein
the medical apparatus system (1) comprises marking components, guiding components (17) or docking components attachable on a floor of a predefined room for positioning the medical apparatus-side interface component (10") of the mobile component of the medical apparatus (2) with respect to the room-side interface component (10').

3. The medical apparatus system (1) of any preceding claim, wherein
the room-side interface component (10') comprises a charging electronics (18) configured to control the charging operation.

4. The medical apparatus system (1) of claim 3, wherein
the charging electronics (18) is configured to automatically start the charging operation after a connection of the room-side interface component (10') and the medical apparatus-side interface component (10").

5. The medical apparatus system (1) of any preceding claim, wherein
the mobile component of the medical apparatus (2) is formed by an operating table.

6. The medical apparatus system (1) of anyone of claims 1 to 4, wherein
the mobile component of the medical apparatus (2) is formed by a lamp.

7. A method for charging an accumulator (7) of a mobile component of a medical apparatus (2) of a medical apparatus system (1) of any preceding claim, comprising the steps:
positioning the medical apparatus-side interface component (10") of the mobile component of the medical apparatus (2) with respect to the room-side interface component (10');
automatically connecting the room-side interface component (10') and the medical apparatus-side interface component (10"); and
charging the accumulator (7) of the mobile component of the medical apparatus (2) by transmitting electric power via the room-side interface component (10') and the medical apparatus-side interface component (10").

8. Method of claim 7 including the step:
automatically starting charging of the accumulator (7) after connecting the interface components (10', 10").

9. Method of claim 7 or 8, wherein
the positioning is performed by aligning specific features of the mobile component of the medical apparatus (2) and markings, guiding components (17), or docking components on a floor of a predefined room.

10. Method of anyone of claims 7 to 9, wherein
charging is performed by an inductive connection of the interface components.

## Patentansprüche

1. Medizingerätesystem (1), das aufweist:
eine mobile Komponente eines Medizingeräts (2), das einen integrierten Akkumulator (7) zum Bereitstellen von elektrischer Energie für eine Steuerungseinrichtung (5) und für elektrische Aktuatoren (4) der mobilen Komponente des Medizingeräts (2) aufweist, und
eine Schnittstelle (10), die dazu ausgebildet ist, nur an einer ortsfesten Verbindungsstelle, eine Stromversorgungskomponente (9) und den Akkumulator (7) automatisch zu verbinden, wobei
die Schnittstelle (10) eine raumseitige Schnittstellenkomponente (10') und eine zusammenarbeitende medizingeräteseitige Schnittstellenkomponente (10") aufweist,
wobei
die raumseitige Schnittstellenkomponente (10') eine Unterflurkomponente und eine oder mehrere in einem Boden angeordnete erste Induktionsspulen aufweist,
und
die geräteseitige Schnittstellenkomponente (10") in Tragkonstruktionen (14) der mobilen Komponente des Medizingeräts (2) angeordnet ist,
**dadurch gekennzeichnet, dass**
die mobile Komponente des Medizingeräts (2) Aufbockstempel als die Tragkonstruktionen (14) zum Absichern eines sicheren Stands der mobilen Komponente des Medizingeräts (2) aufweist, und
eine oder mehrere zweite Induktionsspulen (13) an den Tragkonstruktionen (14) angebracht sind.

2. Medizingerätesystem (1) nach Anspruch 1, wobei
das Medizingerätesystem (1) auf einem Boden eines vorbestimmten Raums anbringbare Markierungskomponenten, Führungskomponenten (17) oder Andockkomponenten aufweist, um die medizingeräteseitige Schnittstellenkomponente (10") der mobilen Komponente des Medizingeräts (2) bezüglich der raumseitigen Schnittstellenkomponente (10') zu positionieren.

3. Medizingerätesystem (1) nach einem der vorangehenden Ansprüche, wobei
die raumseitige Schnittstellenkomponente (10') eine Ladeelektronik (18) aufweist, die dazu ausgebildet ist, die Ladeoperation zu steuern.

4. Medizingerätesystem (1) nach Anspruch 3, wobei
die Ladelektronik (18) dazu ausgebildet ist, die Ladeoperation nach einem Verbinden der raumseitigen Schnittstellenkomponente (10') und der medizingeräteseitigen Schnittstellenkomponente (10") automatisch zu starten.

5. Medizingerätesystem (1) nach einem der vorangehenden Ansprüche, wobei
die mobile Komponente des Medizingeräts (2) durch einen Operationstisch gebildet ist.

6. Medizingerätesystem (1) nach einem der Ansprüche 1 bis 4, wobei die mobile Komponente des Medizingeräts (2) durch eine Lampe gebildet ist.

7. Verfahren zum Laden eines Akkumulators (7) einer mobilen Komponente eines Medizingeräts (2) eines Medizingerätsystems (1) nach einem der vorangehenden Ansprüche, das die Schritte aufweist:
Positionieren der medizingeräteseitigen Schnittstellenkomponente (10") der mobilen Komponente des Medizingeräts (2) bezüglich der raumseitigen Schnittstellenkomponente (10');
Automatisches Verbinden der raumseitigen Schnittstellenkomponente (10') und der medizingeräteseitigen Schnittstellenkomponente (10"); und
Aufladen des Akkumulators (7) der mobilen Komponente des Medizingeräts (2) durch Übertragen von elektrischer Energie über die raumseitige Schnittstellenkomponente (10') und die medizingeräteseitige Schnittstellenkomponente (10").

8. Verfahren nach Anspruch 7, das den Schritt enthält:
Automatisches Starten eines Ladens des Akkumulators (7) nach einem Verbinden der Schnittstellenkomponenten (10', 10").

9. Verfahren nach Anspruch 7 oder 8, wobei das Positionieren durch ein Ausrichten von spezifischen Merkmalen der mobilen Komponente des Medizingeräts (2) und Markierungen, Führungskomponenten (17) oder Andockkomponenten auf einem Boden eines vorbestimmten Raums durchgeführt wird.

10. Verfahren nach einem der Ansprüche 7 bis 9, wobei
Aufladen durch eine induktive Verbindung der Schnittstellenkomponenten durchgeführt wird.

## Revendications

1. Système d'appareil médical (1) comprenant :
un composant mobile d'un appareil médical (2) comprenant un accumulateur intégré (7) pour fournir de l'énergie électrique pour un dispositif de commande (5) et pour des actionneurs électriques (4) du composant mobile de l'appareil médical (2) ; et
une interface (10) configurée de manière à connecter automatiquement un dispositif d'alimentation électrique (9) et l'accumulateur (7) uniquement à un emplacement de connexion stationnaire, dans lequel
l'interface (10) comprend un composant d'interface côté pièce (10') et un composant d'interface côté appareil médical collaborant (10") ;
dans lequel
le composant d'interface côté pièce (10') comprend un composant sous plancher et une ou plusieurs premières bobines d'induction situées dans un plancher ; et
le composant d'interface côté appareil (10") est situé dans des structures de support (14) du composant mobile de l'appareil médical (2) ;
**caractérisé en ce que**
le composant mobile de l'appareil médical (2) comprend des tampons autoélévateurs en tant que les structures de support (14) pour assurer une mise en place sûre du composant mobile de l'appareil médical (2) ;
une ou plusieurs secondes bobines d'induction (13) sont agencées au niveau des structures de support (14).

2. Système d'appareil médical (1) selon la revendication 1, dans lequel :
le système d'appareil médical (1) comprend des composants de marquage, des composants de guidage (17) ou des composants d'amarrage pouvant être fixés sur un plancher d'une pièce prédéfinie en vue de positionner le composant d'interface côté appareil médical (10") du composant mobile de l'appareil médical (2) par rapport au composant d'interface côté pièce (10').

3. Système d'appareil médical (1) selon l'une quelconque des revendications précédentes, dans lequel :
le composant d'interface côté pièce (10') comprend un équipement électronique de charge (18) configurée de manière à commander l'opération de charge.

4. Système d'appareil médical (1) selon la revendication 3, dans lequel :
l'équipement électronique de charge (18) est configuré de manière à démarrer automatiquement l'opération de charge après une connexion du composant d'interface côté pièce (10') et du composant d'interface côté appareil médical (10").

5. Système d'appareil médical (1) selon l'une quelconque des revendications précédentes, dans lequel :
le composant mobile de l'appareil médical (2) est formé par une table d'opération.

6. Système d'appareil médical (1) selon l'une quelconque des revendications 1 à 4, dans lequel :
le composant mobile de l'appareil médical (2) est formé par une lampe.

7. Procédé de charge d'un accumulateur (7) d'un composant mobile d'un appareil médical (2) d'un système d'appareil médical (1) selon l'une quelconque des revendications précédentes, comprenant les étapes ci-dessous consistant à :
positionner le composant d'interface côté appareil médical (10") du composant mobile de l'appareil médical (2) par rapport au composant d'interface côté pièce (10') ;
connecter automatiquement le composant d'interface côté pièce (10') et le composant d'interface côté appareil médical (10") ; et
charger l'accumulateur (7) du composant mobile de l'appareil médical (2) en transmettant de l'énergie électrique par l'intermédiaire du composant d'interface côté pièce (10') et du composant d'interface côté appareil médical (10").

8. Procédé selon la revendication 7, comprenant l'étape ci-dessous consistant à :
démarrer automatiquement la charge de l'accumulateur (7) après avoir connecté les composants d'interface (10', 10").

9. Procédé selon la revendication 7 ou 8, dans lequel :
l'étape de positionnement est mise en œuvre en alignant des caractéristiques spécifiques du composant mobile de l'appareil médical (2) et des marquages, des composants de guidage (17), ou des composants d'amarrage sur un plancher d'une pièce prédéfinie.

10. Procédé selon l'une quelconque des revendications 7 à 9, dans lequel :
l'étape de charge est mise en œuvre par une connexion inductive des composants d'interface.
